(19) Europäisches Patentamt
European Patent Office
Office européen des brevets

(11) Publication number: **0 023 938**
**B1**

# (12) EUROPEAN PATENT SPECIFICATION

(45) Date of publication of patent specification: **20.07.83**

(51) Int. Cl.$^3$: **C 07 D 207/32**

(21) Application number: **79301631.2**

(22) Date of filing: **13.08.79**

## (54) Preparation of pyrrole-2-acetates.

(43) Date of publication of application:
**18.02.81 Bulletin 81/7**

(45) Publication of the grant of the patent:
**20.07.83 Bulletin 83/29**

(84) Designated Contracting States:
**AT BE CH DE FR GB IT NL SE**

(56) References cited:
**DE - A - 2 302 671**
**US - A - 3 752 826**
**US - A - 3 957 818**

**MONATSHEFTE FÜR CHEMIE, 100. Band, 1969, pages 724—733 Springer-Verlag New York, U.S.A. F. ASINGER et al.: "Über die Synthese von N,N'-disubstituierten alpha-Iminophenylglyoxylsäurethionamiden und deren Reduktion mit Schwefelwasserstoff zu Phenylessigsäurethionamiden"**
**Reaktionen der Organischen Chemie, Alfred Hüthig Verlag, P. 541.**

(73) Proprietor: **McNeilab, Inc.**
**Camp Hill Road**
**Fort Washington Pennsylvania 19034 (US)**

(72) Inventor: **Carson, John Robert**
**551 Rittenhouse Boulevard**
**Norristown, Pennsylvania (US)**
Inventor: **Carmosin, Richard J.**
**341-B Willowbrook Drive**
**Norristown, Pennsylvania (US)**
Inventor: **Stefanski, Anthony T.**
**1550 Irene Street, Apt. 617**
**Bethlehem, Pennsylvania (US)**

(74) Representative: **Grundy, Derek George Ritchie et al,**
**CARPMAELS & RANSFORD 43, Bloomsbury Square**
**London WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 023 938 B1

# Preparation of pyrrole-2-acetates

This invention relates to a novel process of preparing loweralkyl 1-methylpyrrole-2-acetates of the formula:

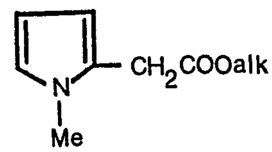

(I)

wherein alk is loweralkyl, preferably ethyl.

According to the instant process, an appropriate loweralkyl $\alpha$-imino-1-methylpyrrole-2-acetate of formula (II), wherein alk is as previously defined and R is hydrogen, loweralkyl, cycloalkyl, phenyl or phenyl substituted with from one to three substituents each selected from loweralkyl, loweralkyloxy and halo, is reduced to a corresponding loweralkyl 1-methyl-pyrrole-2-acetate of formula (I) by the action of a sulfur-containing reducing agent.

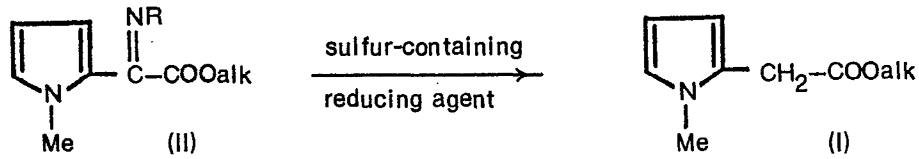


(II) (I)

As used herein, "loweralkyl" refers to straight or branch chained alkyls having from 1 to 6 carbons, for example, methyl, ethyl, propyl, isopropyl, butyl, *tert*-butyl, pentyl, hexyl and the like; "halo" refers to chloro, bromo, fluoro and iodo; and "cycloalkyl" refers to cyclopentyl and cyclohexyl.

Sulfur-containing reducing agents preferred for the foregoing reduction reaction are hydrogen sulfide, an alkali metal hydrogen sulfide, e.g., sodium hydrogen sulfide, sodium dithionite and the like. Also suitable are sodium polysulfide and ammonium polysulfide. Most preferred is hydrogen sulfide.

Suitable solvents which may be utilized for the reduction reaction are aromatic hydrocarbons such as, for example, benzene, toluene, xylene and the like; halacarbons such as, for example, methylene dichloride, chloroform, and the like; amines such as, for example, pyridine, triethylamine, triethanolamine and the like; lower alkanols, such as, for example, methanol, ethanol, methoxyethanol and the like; and dipolar aprotic organic solvents such as, for example, dimethyl sulfoxide (DMSO), N,N-dimethylformamide (DMF), ethyl acetate, acetonitrile and the like.

The reduction reaction may be performed at reaction temperatures varying from −20°C. to 50°C. and atmospheric pressure to 60 p.s.i. ($4.2 \times 10^4$ $Kg/m^2$) pressure may be advantageously employed.

The loweralkyl 1-methylpyrrole-2-acetates of formula (I) have been reported in the literature as being useful intermediates in the preparation of 5-aroyl-pyrrole-2-acetic acid derivatives having antiinflammatory activity (e.g., see U.S. Patent Nos. 3,752,826; 3,803,169; 3,846,447; and 3,957,818).

The $\alpha$-imino-acetate precursors of formula (II) are obtainable by several methods. For example, by reacting a loweralkyl 1-methylpyrrole-2-glyoxylate of formula (III) wherein alk is as previously defined, with an appropriate phenylamine of formula (IV), wherein n is 0 or an integer from 1 to 3 and each X is hydrogen, loweralkyl, loweralkyloxy or halo, the corresponding $\alpha$-imino-acetates of formula (II) wherein R is phenyl or substituted phenyl are obtained (II-a). The reaction may be conducted in an anhydrous inert organic solvent, such as, for example, the aromatic hydrocarbons and halocarbons previously described and, preferably, under reflux conditions with azeotropic removal of water formed during the course of the reaction. The presence of a catalytic amount of a strong acid, e.g., an organic acid such as p-toluene-sulfonic acid, methanesulfonic acid and the like or a mineral acid such as $H_2SO_4$, HCl and the like, may be employed to enhance the rate or reaction.

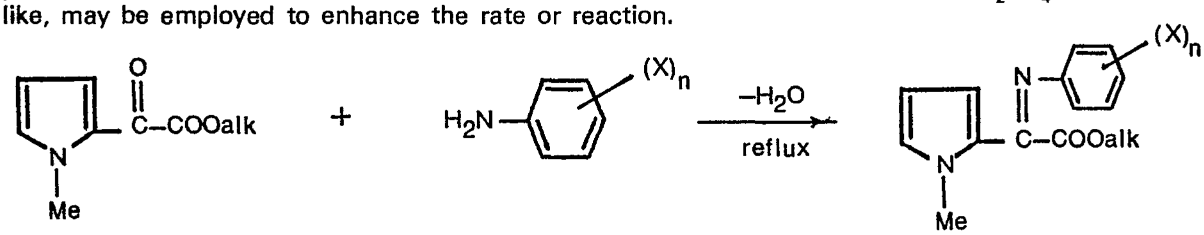


(III) (IV) (II–a)

The $\alpha$-imino-acetates of formula (II) wherein R is loweralkyl or cycloalkyl (II-b) may be prepared by reacting the glyoxylate of formula (III) with a stoichiometric excess of an appropriate alkylamine of formula (V), wherein Y is loweralkyl or cycloalkyl, in an aprotic organic solvent under an inert

atmosphere, e.g., nitrogen, argon and the like and in the presence of titanium tetrachloride. At least six molar equivalents of (V) to one molar equivalent of (III) is preferred. Suitable solvents include aromatic hydrocarbons, e.g., benzene, toluene, xylene and the like; ethers, e.g., diethyl ether, dioxane, tetrahydrofuran and the like; and halogenated hydrocarbons, e.g., methylene dichloride, chloroform and the like. The reaction is preferably conducted in the cold, e.g., at temperatures from −20°C. to 10°C.

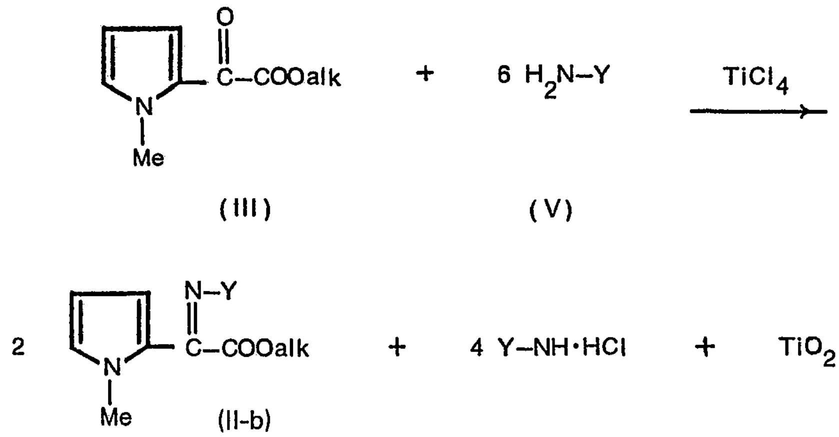


(III) (V) (II-b)

The α-imino-acetates of formula (II) wherein R is hydrogen may be prepared by the interaction of N-methylpyrrole (VI) and loweralkyl cyanoformate (VII) in the presence of hydrogen chloride under Houben Hoesch reaction conditions. In general, dry HCl gas is bubbled through a solution of (VI) and (VII) in an aprotic organic solvent suitable for Houben Hoesch reactions, e.g., ethers, halogenated hydrocarbons, aromatic hydrocarbons, and the like. Alternatively, an ethereal solution of HCl is slowly added to the solution of (VI) and (VII). The resultant loweralkyl α-imino-1-methylpyrrole-2-acetate HCl salt (VIII) is transformed to the corresponding free imino state (II—c) by treatment with at least an equivalent amount of a suitable base, e.g., an alkali metal carbonate or bicarbonate, or a liquid amine which can serve as a halogen acid acceptor such as pyridine, triethylamine and the like.

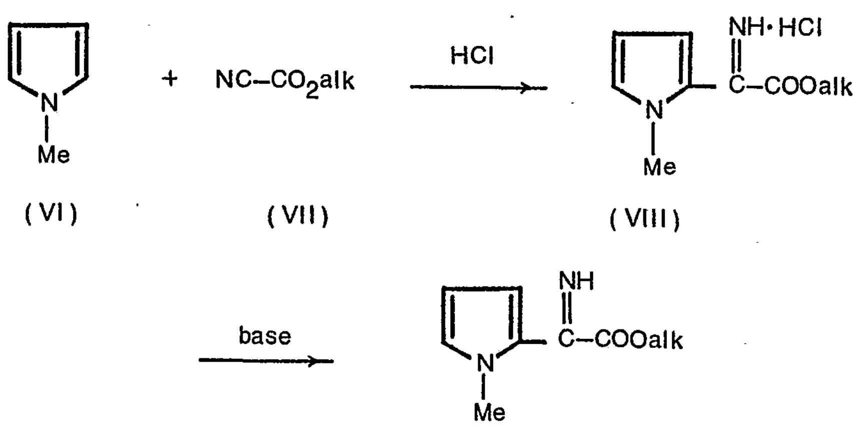


(VI) (VII) (VIII)

(II—c)

The loweralkyl 1-methylpyrrole-2-glyoxylates of formula (III) may be prepared according to the method described by A. Treibs and F. H. Kreuzer, *Ann., 721,* 105 (1969). The loweralkyl cyanoformates of formula (VII) may be prepared in accordance with the method described by M. E. Childs and W. P. Weber, *J. Org. Chem., 41,* 3486 (1976).

The α-imino-acetates of formulas (II-a), (II-b) and (II-c), collectively represented by formula (II), are subsequently reduced to the desired loweralkyl 1-methyl-pyrrole-2-acetates of formula (I) using a sulfur-containing reducing agent as previously described.

Example I

A. *Ethyl 1-methylpyrrole-2-acetate:* A stream of dry hydrogen chloride is passed at a moderate rate through a solution of 12.5 g. of N-methylpyrrole and 16.8 g. of ethyl cyanoformate in 125 mls. of ethanol-free chlorform at 22°C. for 3½ hours. The reaction mixture is added dropwise with stirring to a 1.5 liter solution of 5% sodium bicarbonate. The chloroform layer is separated, dried over $Na_2SO_4$ and

evaporated *in vacuo* at room temperature to give a brown oil (ethyl α-imino-1-methylpyrrole-2-acetate). The oil is dissolved in 125 ml. of ethanol. Hydrogen sulfide is passed through the solution vigorously for 45 mins. at 22°C. The mixture is allowed to stand for 16 hrs. The solvent is evaporated *in vacuo*. The residue is taken up in chloroform. Sulfur is removed by filtration. The chloroform is evaporated *in vacuo*. The residue is distilled *in vacuo* to give 15.5 g. (60% yield) of oily ethyl 1-methylpyrrole-2-acetate, b.p. 64—66°C. at 0.06 Torr.

B. The procedure of Example I—A is followed except that an equivalent amount of methyl cyanoformate and n-butyl cyanoformate are substituted for the ethyl cyanoformate used therein to yield, as respective products:

methyl 1-methylpyrrole-2-acetate; and
*n*-butyl 1-methylpyrrole-2-acetate.

Example II

A. *Ethyl α-imino-1-methylpyrrole-2-acetate hydrochloride:* A stream of dry hydrogen chloride is bubbled slowly into a solution of 2.7 g. of ethyl cyanoformate and 2.0 g. of N-methylpyrrole in 20 ml. of ethanol-free chloroform at 22°C. for $5\frac{3}{4}$ hours. The imine hydrochloride is measured by hydrolyzing for 30 mins. with water and determining the hydrolysis product, ethyl 1-methylpyrrole-2-glyoxylate, gas chromatographically with internal standard. A 75% yield is measured.

B. Following the procedure above but substituting toluene and tetrahydrofuran for chloroform, yields of 65% and 70%, respectively, are obtained.

Example III

*Ethyl-1-methylpyrrole-2-acetate:* A 28 ml. solution of ethereal hydrogen chloride (.049 mole of HCl) is added dropwise over a 3 hr. period to a solution of 2.1 g. of N-methylpyrrole and 2.9 g. of ethyl cyanoformate in 3 ml. ether. The ether supernatant is decanted off, and the precipitate taken up in 11 mls. of dry pyridine and the mixture transferred to a pressure bottle. Hydrogen sulfide (13 g.) is added to the mixture at −78°C., the bottle sealed and the mixture is stirred at ambient temperature overnight (about 16 hours). Gas chromatographic analysis with internal standard shows 57% yield of ethyl N-methylpyrrole-2-acetate.

Example IV

*Ethyl-1-methylpyrrole-2-acetate:* Dry hydrogen chloride gas is bubbled slowly into a solution of N-methylpyrrole (5 g.) and ethyl cyanoformate (6.72 g.) in 50 ml. of dry spectral grade chloroform for 3.3 hrs. at 25°C. The excess hydrogen chloride and a portion of the chloroform are evaporated off; 50 ml. of 2-methoxyethanol is added; and the remaining chloroform evaporated under vacuum. Then, 25 ml. of dry triethylamine is added and hydrogen sulfide is vigorously bubbled through the solution for 30 mins. Gas chromatographic analysis with internal standard shows 50% yield of ethyl 1-methylpyrrole-2-acetate.

Example V

*Ethyl-1-methylpyrrole-2-acetate:* Dry hydrogen chloride is bubbled slowly into a solution of N-methylpyrrole (1 g.) and ethyl cyanoformate (1.35 g.) in dry spectral grade chloroform (10 ml.) for 4 hrs. at 25°C. The solution is then added dropwise to 10 ml. of triethanolamine with simultaneous introduction of hydrogen sulfide gas bubbled through the solution over a 1.5 hr. period. Gas chromatographic analysis with internal standard shows 61% yield of ethyl 1-methylpyrrole-2-acetate.

Example VI

*Ethyl α-(4-methoxyphenylimino)-1-methylpyrrole-2-acetate:* To a solution of 4.0 g. ethyl 1-methylpyrrole-2-glyoxylate and 2.96 g. *p*-anisidine in 20 ml. toluene is added 24 mg. *p*-toluenesulfonic acid. Heating at reflux for 4 days with water being removed with a Dean-Stark trap is followed by washing successively with aqueous hydrochloric acid, aqueous sodium bicarbonate and brine. After drying, evaporation of the solvent affords a dark solid. Recrystallization from isopropanol gives a 58% yield of ethyl α-(4-methoxyphenylimino)-1-methylpyrrole-2-acetate, m.p. 83—85°C.

Example VII

*Ethyl 1-methylpyrrole-2-acetate:* Sodium ethoxide, 1.14 g., is added to 0.57 g. hydrogen sulfide dissolved in 50 ml. ethanol. A portion of this solution, 4.12 ml., is added to 0.20 g. ethyl α-(4-methoxyphenylimino)-1-methylpyrrole-2-acetate and stirred 2 hrs. Analysis by gas chromatography with an internal standard measures 29% ethyl 1-methylpyrrole-2-acetate.

Example VIII

*Ethyl α-cyclohexylimino-1-methylpyrrole-2-acetate:* To a solution of 25 g. ethyl 1-methylpyrrole-2-glyoxylate and 44.5 g. cyclohexylamine in 700 ml. ether under nitrogen at 0°C is added 8.8 ml. of titanium tetrachloride in 80 ml. pentane. After stirring at room temperature overnight, the precipitate is removed by filtration. The filtrate is evaporated and the resulting oil distilled. The fraction collected at

111—117°C. (5 millitorr) is crystallized from hexane to afford a 15% yield of ethyl $\alpha$-cyclohexylimino-1-methylpyrrole-2-acetate, m.p. 35—36°C.

Example IX

*Ethyl $\alpha$-phenylimino-1-methylpyrrole-2-acetate:* To a solution of 4.0 g. ethyl 1-methylpyrrole-2-glyoxylate and 2.23 g. aniline in 20 ml. toluene is added 30 mg. *p*-toluenesulfonic acid. Heating at reflux for 2 days with water being removed with a Dean-Stark trap is followed by dilution with ether. The precipitate is removed by filtration and the filtrate washed successively with aqueous hydrochloric acid, aqueous sodium bicarbonate and brine. After drying, the solvent is evaporated to afford a residue which is recrystallized twice from isopropanol to give a 43% yield of ethyl $\alpha$-phenylimino-1-methylpyrrole-2-acetate, m.p. 69—71°C.

Example X

*Ethyl 1-methylpyrrole-2-acetate:* Three equivalents of sodium dithionite (0.36 g.) is added in one portion with vigorous stirring to a solution of 0.2 g. ethyl $\alpha$-(4-methoxyphenylimino)-1-methylpyrrole-2-acetate in 6 ml. glacial acetic acid plus 2.5 ml. water. After ten minutes, two additional equivalents of dithionite is added. After 10 mins. more, a sufficient sample for G.C. analysis is poured into ice and extracted with chloroform. Analysis by gas chromatography (G.C.) with an internal standard measures 34% ethyl 1-methylpyrrole-2-acetate.

Example XI

A. *Ethyl 1-methylpyrrole-2-acetate:* Ten equivalents (0.7 g.) of hydrogen sulfide is added to a solution of 0.60 g. ethyl $\alpha$-(4-methoxyphenylimino)-1-methylpyrrole-2-acetate in 38 ml. dimethyl sulfoxide at −78°C. in a pressure bottle. The bottle is sealed and the solution is stirred at room temperature for 4 hours. Excess hydrogen sulfide is vented and the mixture is diluted with chloroform. Analysis by gas chromatography with an internal standard measures 97% ethyl 1-methylpyrrole-2-acetate.

B. Using the above procedure, a solution of ethyl $\alpha$-phenylimino-1-methylpyrrole-2-acetate in 6.0 ml. methanol produces 74% product. Likewise, a solution of ethyl $\alpha$-cyclohexylimino-1-methylpyrrole-2-acetate in 2.5 ml. methanol produces 88% product.

Example XII

A. By following the procedure of Example VI, except that an equivalent amount of 4-chloroaniline, 4-ethoxyaniline, 2-bromo-4-ethylaniline, 2,4-dimethoxyaniline, 2,4,6-trimethylaniline and 2,4,6-trichloroaniline are substituted for the *p*-anisidine used therein, the following respective products are obtained.

ethyl $\alpha$-(4-chlorophenylimino)-1-methylpyrrole-2-acetate;
ethyl $\alpha$-(4-ethoxyphenylimino)-1-methylpyrrole-2-acetate;
ethyl $\alpha$-(2-bromo-4-ethylphenylimino)-1-methylpyrrole-2-acetate;
ethyl $\alpha$-(2,4-dimethoxyphenylimino)-1-methylpyrrole-2-acetate;
ethyl $\alpha$-(2,4,6-trimethylphenylimino)-1-methylpyrrole-2-acetate; and
ethyl $\alpha$-(2,4,6-trichlorophenylimino)-1-methylpyrrole-2-acetate.

B. Each of the foregoing $\alpha$-imino-acetates may be reduced to ethyl 1-methylpyrrole-2-acetate according to the procedure of Example XI—A using $H_2S$ as the reducing agent.

Example XIII

A. The procedure of Example VIII is repeated except that an equivalent quantity of *n*-butylamine and cyclopentylamine is substituted for the cyclohexylamine used therein to yield, as respective products:

ethyl $\alpha$-*n*-butylimino-1-methylpyrrole-2-acetate; and
ethyl $\alpha$-cyclopentylimino-1-methylpyrrole-2-acetate.

B. Reduction of each of the foregoing $\alpha$-imino-acetates to ethyl 1-methylpyrrole-2-acetate with $H_2S$ as the reducing agent may be carried out according to the procedure of Example XI—A.

**Claims**

1. A process of preparing a $C_1$—$C_6$ alkyl 1-methylpyrrole-2-acetate which comprises reducing a $C_2$—$C_6$ alkyl $\alpha$-imino-1-methylpyrrole-2-acetate having the formula:

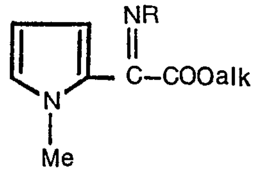

wherein: alk is $C_1$—$C_6$ alkyl; and

R is hydrogen, $C_1$—$C_6$ alkyl, cyclopentyl, cyclohexyl, phenyl or phenyl substituted with from one to three substituents each selected from $C_1$—$C_6$, $C_1$—$C_6$ alkyloxy and halo;
by the action of a sulfur-containing reducing agent.

2. The process of Claim 1, wherein said reducing agent is hydrogen sulfide, sodium hydrogen sulfide or sodium dithionite.

3. A process according to Claim 1 or Claim 2 wherein the $C_1$—$C_6$ alkyl $\alpha$-imino 1-methylpyrrole-2-acetate is prepared by reacting a $C_1$—$C_6$ alkyl 1-methylpyrrole-2-glyoxylate having the formula:

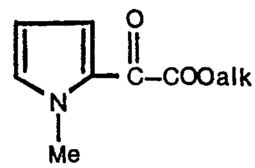

with a phenylamine having the formula:

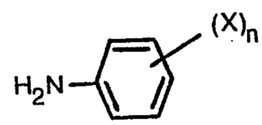

in an anhydrous inert organic solvent in the presence of a catalytic amount of a strong acid with azeotropic removal of water formed during the reaction to form an $\alpha$-imino-acetate having the formula:

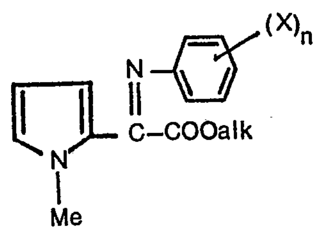

wherein in the foregoing formulas:
alk is $C_1$—$C_6$ alkyl;
n is 0 or an integer from 1 to 3; and
X is hydrogen, $C_1$—$C_6$ alkyl, $C_1$—$C_6$ alkyloxy or halo.

4. The process of Claim 3, wherein said strong acid is $p$-toluenesulfonic acid.

5. A process according to Claim 1 or Claim 2 wherein the $C_1$—$C_6$ alkyl $\alpha$-imino 1-methylpyrrole-2-acetate is prepared by reacting a $C_1$—$C_6$ alkyl 1-methylpyrrole-2-glyoxylate having the formula:

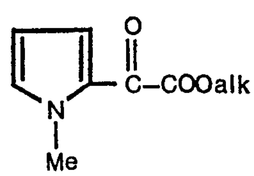

with a stoichiometric excess of an alkylamine having the formula:

$$H_2N—Y$$

in an aprotic organic solvent under an inert atmosphere in the presence of titanium tetrachloride at a temperature of from −20°C. to 10°C. to form an $\alpha$-imino-acetate having the formula:

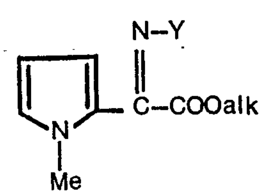

wherein in the foregoing formulas:

alk is $C_1$—$C_6$ alkyl; and

Y is $C_1$—$C_6$ alkyl, cyclopentyl or cyclohexyl.

6. A process according to Claim 1 or Claim 2 wherein the $C_1$—$C_6$ alkyl $\alpha$-imino 1-methylpyrrole-2-acetate is prepared by treating a solution of N-methylpyrrole and $C_1$—$C_6$ alkyl cyanoformate in an aprotic organic solvent with hydrogen chloride to form an $\alpha$-imino-acetate hydrochloride having the formula:

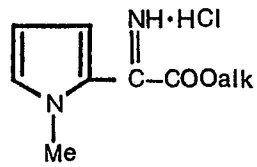

wherein alk is $C_1$—$C_6$ alkyl, and reacting said $\alpha$-iminoacetate hydrochloride with base to yield the corresponding $\alpha$-imino-acetate.

7. A process according to any one of Claims 1 to 6 wherein ethyl 1-methylpyrrole-2-acetate (alk=ethyl) is prepared.

8. A process according to Claim 1 or Claim 2 wherein alk is ethyl and the ethyl $\alpha$-imino 1-methylpyrrole-2-acetate is prepared by treating a solution of N-methylpyrrole and ethyl cyanoformate in an aprotic organic solvent with hydrogen chloride to form ethyl $\alpha$-imino-1-methylpyrrole-2-acetate hydrochloride, and the latter is reacted with an equivalent amount of base.

**Revendications**

1. Un procédé pour préparer un 1-méthylpyrrole-2-acétate d'alkyle en C1—C6 qui consiste à réduire un alphaimino-1-méthyl-pyrrole-2-acétate d'alkyle en C1—C6 répondant à la formule

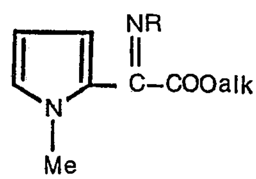

dans laquelle alk représente un groupe alkyle en C1—C6 et R représente l'hydrogène, un groupe alkyle en C1—C6, cyclopentyle, cyclohexyle, phényle ou phényle portant 1 à 3 substituants choisis parmi les groupes alkyles en C1—C6, alkyloxy en C1—C6 et les halogènes, à l'aide d'un agent réducteur contenant du soufre.

2. Procédé selon la revendication 1, caractérisé en ce que l'agent réducteur est le sulfure d'hydrogène, le sulfhydrate de sodium ou l'hydrosulfite de sodium.

3. Un procédé selon la revendication 1 ou 2 dans lequel l'alpha-imino-1-méthylpyrrole-2-acétate d'alkyle en C1—C6 est préparé en faisant réagir un 1-méthylpyrrole-2-glyoxylate d'alkyle en C1—C6 répondant à la formule:

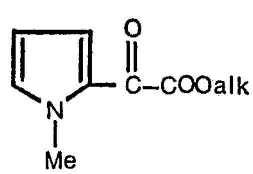

avec une phénylamine répondant à la formule:

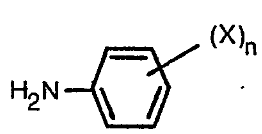

dans un solvant organique inerte anhydre en présence d'une quantité catalytique d'un acide fort avec élimination azéotropique de l'eau formée dans la réaction, la réaction donnant un alpha-imino-acétate de formule:

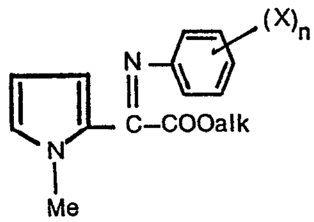

les symboles des formules ci-dessus ayant les significations suivantes:

alk représente un groupe alkyle en C1—C6,

n est égal à 0 ou représente un nombre de 1 à 3 et

X est l'hydrogène, un groupe alkyle en C1—C6, alkoxy en C1—C6 ou un halogène.

4. Procédé selon la revendication 3, caractérisé en ce que l'acide fort est l'acide p-toluène-sulfonique.

5. Procédé selon la revendication 1 ou 2, dans lequel l'alpha-imino-1-méthylpyrrole-2-acétate d'alkyle en C1—C6 est préparé en faisant réagir un 1-méthylpyrrole-2-glyoxylate d'alkyle en C1—C6 répondant à la formule:

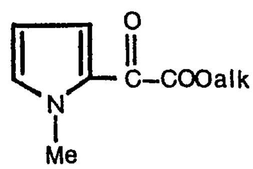

avec un excès par rapport à la quantité stoechiométrique d'une alkylamine répondant à la formule:

$$H_2N—Y$$

dans un solvant organique aprotonique en atmosphère de gaz inerte en présence de tétrachlorure de titane à une température de —20 à 10°C, la réaction donnant un alphaimino-acétate de formule:

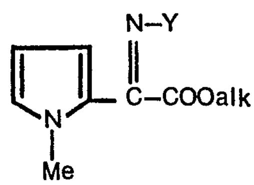

les symboles utilisés dans les formules ci-dessus ayant les significations suivantes:

alk représente un groupe alkyle en C1—C6 et

Y représente une groupe alkyle en C1—C6, cyclopentyle ou cyclohexyle.

6. Procédé selon la revendication 1 ou 2, dans lequel l'alpha-imino-1-méthylpyrrole-2-acétate d'alkyle en C1—C6 est préparé en traitant une solution de N-méthylpyrrole et d'un cyanoformiate d'alkyle en C1—C6 dans un solvant organique aprotonique par le chlorure d'hydrogène, la réaction donnant un chlorhydrate d'alpha-imino-acétate répondant à la formule

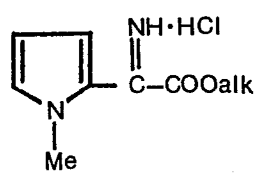

dans laquelle alk représente un groupe alkyl en C1—C6, qu'on fait réagir avec une base pour obtenir l'alpha-iminoacétate correpondant.

7. Un procédé selon l'une quelconque des revendications 1 à 6, dans lequel on prépare le 1-méthylpyrrole-2-acétate d'éthyle (alk=éthyle).

8. Un procédé selon la revendication 1 ou 2 dans lequel alk représente le groupe éthyle et l'alpha-imino-1-méthylpyrrole-2-acétate d'éthyle est préparé en traitant une solution de N-méthylpyrrole et de cyanoformiate d'éthyle dans un solvant organique aprotonique par du chlorure d'hydrogène, ce qui donne le chlorhydrate de alpha-imino-1-méthylpyrrole-2-acétate d'éthyle qu'on fait réagir avec la quantité équivalente d'une base.

**Patentansprüche**

1. Ein Verfahren zur Herstellung eines $C_1$—$C_6$-Alkyl-1-methylpyrrol-2-acetats, umfassend das Reduzieren eines $C_1$—$C_6$-Alkyl-α-imino-1-methylpyrrol-2-acetats mit der Formel:

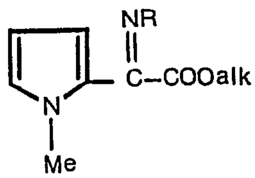

worin alk für $C_1$—$C_6$-Alkyl steht und

R Wasserstoff, $C_1$—$C_6$-Alkyl, Cyclopentyl, Cyclohexyl, Phenyl oder mit einem bis 3 Substituenten, jeweils ausgewählt aus $C_1$—$C_6$-Alkyl, $C_1$—$C_6$-Alkyloxy und Halogen, substituiertes Phenyl darstellt; unter der Einwirkung eines Schwefel enthaltenden Reduktionsmittels.

2. Das Verfahren nach Anspruch 1, worin das genannte Reduktionsmittel Schwefelwasserstoff, Natriumhydrogensulfid oder Natriumdithionit ist.

3. Ein Verfahren nach Anspruch 1 oder 2, worin das $C_1$—$C_6$-Alkyl-α-imino-1-methylpyrrol-2-acetat hergestellt wird durch Umsetzen eines $C_1$—$C_6$-Alkyl-1-methylpyrrol-2-glyoxylats mit der Formel:

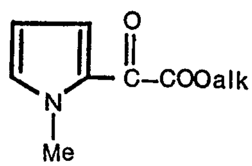

mit einem Phenylamin mit der Formel:

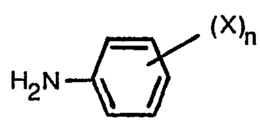

in einem wasserfreien inerten organischen Lösungsmittel in Gegenwart einer katalytischen Menge einer starken Säure unter azeotroper Entfernung von während der Umsetzung gebildetem Wasser zur Ausbildung eines α-Iminoacetats mit der Formel:

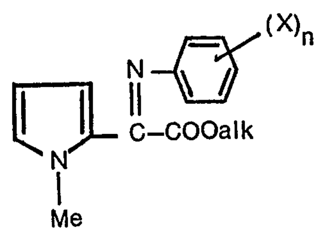

wobei in den vorstehenden Formeln

alk für $C_1$—$C_6$-Alkyl steht;

n für Null oder eine ganze Zahl von 1 bis 3 steht; und

X Wasserstoff, $C_1$—$C_6$-Alkyl, $C_1$—$C_6$-Alkyloxy oder Halogen darstellt.

4. Das Verfahren nach Anspruch 3, worin die genannte starke Säure p-Toluolsulfonsäure ist.

5. Ein Verfahren nach Anspruch 1 oder 2, worin das $C_1$—$C_6$-Alkyl-α-imino-1-methylpyrrol-2-acetate hergestellt wird durch Umsetzen eines $C_1$—$C_6$-Alkyl-1-methylpyrrol-2-glyoxylats mit der Formel:

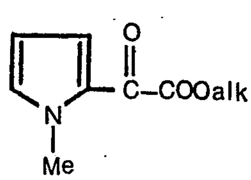

mit einem stöchiometrischen Überschuß eines Alkylamins mit der Formel:

$$H_2N—Y$$

in einem aprotischen organischen Lösungsmittel unter inerter Atmosphäre in Gegenwart von Titantetrachlorid bei einer Temperatur von −20°C bis 10°C unter Ausbildung eines $\alpha$-Iminoacetats mit der Formel:

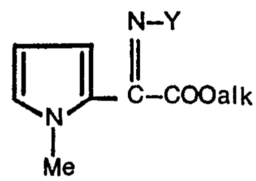

wobei in den vorstehenden Formeln

alk für $C_1$—$C_6$-Alkyl steht; und

Y $C_1$—$C_6$-Alkyl, Cyclopentyl oder Cyclohexyl darstellt.

6. Ein Verfahren nach Anspruch 1 oder 2, worin das $C_1$—$C_6$-Alkyl-$\alpha$-imino-1-methylpyrrol-2-acetat hergestellt wird durch Behandeln einer Lösung von N-Methylpyrrol und $C_1$—$C_6$-Alkylcyanoformiat in einem aprotischen organischen Lösungsmittel mit Chlorwasserstoff unter Ausbildung eines $\alpha$-Iminoacetathydrochlorids mit der Formel:

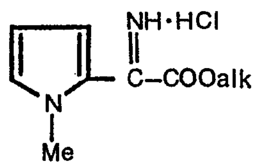

worin alk für $C_1$—$C_6$-Alkyl steht, und durch Umsetzen des genannten $\alpha$-Iminoacetathydrochlorids mit einer Base zur Ausbildung des entsprechenden $\alpha$-Iminoacetats.

7. Ein Verfahren nach einem der Ansprüche 1 bis 6, worin Ethyl-1-methylpyrrol-2-acetat (alk=Ethyl) hergestellt wird.

8. Ein Verfahren nach Anspruch 1 oder 2, worin alk für Ethyl steht und das Ethyl-$\alpha$-imino-1-methylpyrrol-2-acetat hergestellt wird durch Behandlung einer Lösung von N-Methylpyrrol und Ethylcyanoformiat in einem aprotischen organischen Lösungsmittel mit Chlorwasserstoff zur Ausbildung von Ethyl-$\alpha$-imino-1-methylpyrrol-2-acetat-hydrochlorid und Umsetzen des letztgenannten mit einer äquivalenten Menge einer Base.